# EUROPÄISCHE PATENTSCHRIFT

(11) **EP 2 476 375 B1**
(45) Veröffentlichungstag und Bekanntmachung des Hinweises auf die Patenterteilung: **07.06.2017**
(21) Anmeldenummer: 11003336.2
(22) Anmeldetag: 20.04.2011
(51) Int. Cl.: A61B 6/04, A61B 90/14

(54) **Maske und Lagerungsvorrichtung für die radiologische Diagnostik und/oder radioonkologische Behandlung**
Mask and storage device for radiological diagnosis and/or radio-oncological treatment
Masque et dispositif de stockage pour le diagnostic radiologique et/ou le traitement radio-oncologique

(30) Priorität: 14.01.2011 DE 202011001492 U
(43) Veröffentlichungstag der Anmeldung: 18.07.2012
(73) Patentinhaber: IT-V Medizintechnik GmbH, 6020 Innsbruck (AT)
(72) Erfinder: Völp, Markus, 6020 Innsbruck (AT)
(74) Vertreter: Herrmann, Uwe

(56) Entgegenhaltungen:
- EP-A1- 1 449 480
- EP-A1- 2 111 798
- DE-U1-202008 006 049
- GB-A- 2 467 160
- US-A- 5 370 117
- US-A- 5 566 681
- US-A- 5 702 406
- US-A- 5 775 337
- US-A- 5 848 449
- US-A1- 2002 038 659
- US-A1- 2005 045 187
- US-A1- 2005 284 490

## Beschreibung

Die Erfindung betrifft eine Maske zur Fixierung des Kopf- und Schulterbereichs eines Patienten an einer Lagerungsvorrichtung, sowie eine Lagerungsvorrichtung für den Kopf- und Schulterbereich eines Patienten für die radiologische Diagnostik und/oder radioonkologische Behandlung.

Gattungsgemäße Lösungen sind aus dem Stand der Technik bekannt. Sie umfassen typischerweise eine höhen- bzw. positionsverstellbar gelagerte Basisplatte, auf der der Kopf- und Schulterbereich eines Patienten gelagert wird. Zur Fixierung wird eine an die Kopfform des Patienten angepasste Maske verwendet.

Solche Lagerungsvorrichtungen werden unter anderem von der Anmelderin unter dem Produktnamen HeadSTEP vertrieben.

Passende Masken bestehen aus einem thermoplastisch verformbaren Einweg-Maskenmaterial und einem wiederverwertbaren, U-förmigen Rahmen, in den das Maskenmaterial verklemmt wird. Die so entstandene fertige Maske wird erwärmt bis das Maskenmaterial plastisch verformbar ist und über den Kopfbereich des Patienten gezogen, wobei sich das Maskenmaterial an dessen Kopfform anpasst. Die angepasste Maske wird durch Abkühlen ausgehärtet.

Solche Masken werden unter anderem von der Anmelderin unter dem Produktnamen iFrame vertrieben.

Weitere Masken sind unter anderem in der US 2002/0038659 A1, der US 5,775,337 A, der US 2005/0045187 A1 und der GB 2 467 160 A offenbart

Die US 2002/0038659 A1 offenbart eine Maske, in dem ein thermoplastisches Maskenmaterial zwischen einem U-förmigen Rahmen und einem Gegenstück verklemmt wird.

Die US 5,775,337 A offenbart eine Maske mit einer U-förmigen Rahmen, der eine Aufnahme für ein fixierbares Insert aufweist. Ein thermoplastisches Maskenmaterial wird unlösbar an einem solchen Insert fixiert, und der so erhaltene Zusammenschluss an dem Rahmen angebracht. Der Rahmen stellt ein wiederverwertbares Teil dar, während der Zusammenschluss aus Insert und Maskenmaterial nach jedem Patienten entsorgt wird.

Die US 2005/0045187 A1 offenbart eine Maske mit einem U-förmigen Rahmen, der mittels einer Vielzahl an gleichmäßig über den Rahmen verteilten, speziell ausgebildeten Befestigungsstiften an einer Basisplatte arretiert wird.

Die GB 2 467 160 A offenbart eine Maske mit einem U-förmigen Rahmen und einem thermoplastischen Maskenmaterial wobei am thermoplastischen Maskenmaterial Aussparungen vorgesehen sind, um für einen erhöhten Komfort des Patienten zu sorgen.

Bei der Anwendung wird die Maske im Bereich der Schenkelenden und im Bereich der Verbindungsstrebe zwischen den Schenkeln an der Basisplatte arretiert.

Die Einfassung und Befestigung des Maskenmaterials im Rahmen durch den Anwender stellte jedoch eine potentielle Fehlerquelle dar, wodurch die stabile Lagerung des Kopfbereichs beeinträchtigt werden konnte. Zudem kam es wiederholt zu Beschädigungen des Maskenrahmens beim Transport und/oder während der Anwendung. Diese Neigung zu Beschädigungen wurde auch dadurch verstärkt, dass die Rahmen aus dem Stand der Technik aus spröden PAAM-Kunsstoffen gefertigt wurden, da Kunsstoffe mit geeigneteren Materialeigenschaften dazu neigen, sich beim Verklemmen mit dem thermoplastischen Maskenmaterial zu verbinden.

Um diesen Nachteilen entgegenzuwirken wurde ein Einwegrahmen vorgeschlagen, welcher ein fest mit dem Rahmen verbundenes Maskenmaterial aufweist. So konnte die fehlerhafte Einfassung und Befestigung des Maskenmaterials im Rahmen durch den Anwender ausgeschlossen werden. Beschädigungen am Rahmen treten zwar weiterhin auf, jedoch waren durch den Einwegrahmen damit keine signifikanten Kostenfolgen oder Lieferungsverzögerungen mehr verbunden.

Bei der Ausführung der gesamten Maske als Einwegteil ist es vorteilhaft, den Rahmen weniger solide auszuführen. Dabei ergab sich jedoch das Problem, dass der U-förmige Rahmen bedingt durch dessen geringere Festigkeit bei der Anpassung des Maskenmaterials und/oder während der Behandlung im zentralen Bereich der Schenkel durchgebogen wurde und von der Basisplatte abhob. Dadurch war eine optimale und bewegungslose Arretierung des Kopfes des Patienten nicht mehr gewährleistet.

Aufgabe der Erfindung ist, eine Lösung bereitzustellen mit der die eingangs beschriebenen Nachteile von bekannten gattungsgemäßen Systemen behoben werden kann.

Diese Aufgabe wird erfindungsgemäß mit einer Maske gemäß Anspruch 1 gelöst. Vorteilhafte Ausgestaltungen ergeben sich aus den Unteransprüchen.

Demgemäß betrifft die Erfindung eine Maske zur Fixierung des Kopf- und/oder Schulterbereichs eines Patienten an einer Lagerungsvorrichtung für die radiologische Diagnostik und/oder radioonkologische Behandlung. Die Maske umfasst einen U-förmig gestalteten Rahmen und ein thermoplastisch verformbares Maskenmaterial. Erfindungsgemäß weist der U-förmige Rahmen im zentralen Bereich wenigstens eines und vorzugsweise beider Schenkel ein zentrales Arretierungsmittel auf.

Mit Hilfe des zentralen Arretierungsmittels kann der zentrale Bereich der Schenkel des Rahmens der Maske an der Lagerungsvorrichtung arretiert werden. Dadurch wird auch bei einer weniger soliden Ausführung des Maskenrahmens ein Durchbiegen des Rahmens im zentralen Bereich der Schenkel und ein dadurch bedingtes Abheben von der Basisplatte verhindert. Eine optimale und bewegungslose Arretierung des Kopf- und/oder Schulterbereichs der Patienten während der Anpassung des Maskenmaterials und/oder während der Behandlung bleibt auch bei einer vorteilhaften und materialarmen Ausführung von Einwegteilen gewährleistet. So kommen auch die durch die Ausführung der Maske als Einwegteil erreichten Vorteile vollständig und ohne unerwünschten Nebeneffekte zur Geltung.

"Im zentralen Bereich der Schenkel" kann bedeuten, dass die zentralen Arretierungsmittel in einem Abstand von mehr 25% und vorzugsweise mehr als 40% der Gesamtlänge der Schenkel zu sowohl den Schenkelenden als auch der Verbindungsstrebe an den Schenkeln angebracht sind.

Die Schenkel des U-förmigen Rahmens können im Wesentlichen parallel zueinander stehen und/oder gleich lang sein. Eine die Schenkel verbindende Verbindungsstrebe kann ungefähr Im rechten Winkel auf die Schenkel stehen.

Geeignete zentrale Arretierungsmittel umfassen Teile einer Steckverbindung, wie Stifte oder Aufnahmen, oder Aufnahmebereiche für Riegel- und Klammennechaniken.

In einer Ausführungsform besteht die Maske im Wesentlichen aus dem Rahmen und dem Maskenmaterial.

Der Rahmen und das Maskenmaterial sind fest miteinander verbunden. In einer Ausführungsform handelt es sich bei der Maske umfassend den Rahmen und das Maskenmaterial um ein Einwegteil.

"Fest miteinander verbunden" ist so zu verstehen, dass keine reversible Trennung des Rahmens vom Maskenmaterial erfolgen kann, beziehungsweise dass eine solche Trennung zumindest nicht beabsichtigt ist. Rahmen und Maskenmaterial stellen ein integrales Bauteil dar und sind irreversibel miteinander verbunden. Beispiele für eine feste Verbindung umfassen eine durch (beispielsweise thermisches, Laser- oder Ultraschall-) Schweißen erzeugte Verbindung, eine durch Verkleben erzeugte Verbindung, und dergleichen.

In einer Ausführungsform ist der U-förmige Rahmen aus mehreren Teilstücken zusammengesetzt.

In einer Ausführungsform weist der U-förmige Rahmen, vorzugsweise an der Verbindungsstrebe wenigstens ein Gelenk, vorzugsweise ein Drehgelenk auf. Dadurch ist es möglich, die Schenkel des Rahmens gegeneinander aufzuspreizen. In diesem Fall geht die U-Form streng genommen in eine W-Form über, kann jedoch noch als U-Form erkannt und hinreichend zutreffend als solches bezeichnet werden.

In einer Ausführungsform weist die Verbindungsstrebe genau ein Gelenk auf. In einer Ausführungsform ist das Drehgelenk so gestaltet, dass es ausschließlich eine Spreiz- und Schließbewegung in der Ebene des U-förmigen Rahmens ermöglicht.

Der Rahmen der Maske in dieser Ausführungsform kann im gespreizten Zustand über den Kopf des Patienten gezogen werden, anschließend jedoch bei paralleler Schenkelstellung an der Lagerungsvorrichtug arretiert werden. So wird trotz der Fertigung des Rahmens als ein einziges Bauteil und dem damit einhergehenden Stabilitätsgewinn gegenüber der Fertigung aus Teilstücken eine sehr enge Führung des Rahmens um den Kopfbereich des Patienten erreicht. Eine bessere Arretierung durch eine formschlüssige Anpassung des Maskenmaterials an den Kopfbereich des Patienten erreicht werden, als dies andernfalls mit einer zeltartigen Anpassung des Maskenmaterials zwischen den Schenkeln eines U-förmigen Rahmen erreicht werden könnte.

In einer Ausführungsform befindet sich das Gelenk im mittleren Bereich und vorzugsweise in der Mitte der Verbindungsstrebe. "In der Mitte" bedeutet auf halbem Weg zwischen den beiden Schenkeln.

In einer Ausführungsform ist der Rahmen an der Lagerungsvorrichtung ferner im Bereich der Schenkelenden und im Bereich der Verbindungsstrebe arretierbar. Geeignete Arretierungsmittel umfassen Steck- und Schiebeverbindungen, Riegelmechaniken und Klammermechaniken.

In einer Ausführungsform besteht der Rahmen wenigstens teilweise und vorzugsweise im Wesentlichen vollständig aus Polycarbonat. "Im Wesentlichen" bedeutet, dass Teile wie das Gelenk oder die zentralen Arretierungsmittel auch aus einem anderen Material gefertigt sein können, obwohl der Rahmen selbst vollständig aus Polycarbonat besteht.

Polycarbonat hat gegenüber im Stand der Technik verwendeten Rahmenmateriallen wie PAAM den Vorteil, dass es von seinen Materialeigenschaften her besser geeignet, z.B. weniger spröde ist und damit der vorgeschlagene Einwegrahmen trotz seiner gegebenenfalls weniger soliden Ausführung weniger anfällig für Beschädugungen durch einflüsse wie einen Aufprall auf den Boden und dergleichen ist.

In einer Ausführungsform weist der Rahmen zumindest abschnitsweise eine Streben- und/oder Fächerkonstruktion auf, oder ist als Streben- und/oder Fächerkonstruktion gefertigt sein.

Dabei ergibt sich der Vorteil einer vergleichsweise hohen Festigkeit bei geringem Materialbedarf. "Zumindest abschnittsweise" kann beispielsweise bedeuten, dass der Rahmen nur im Bereich der Schenkel und/oder der Verbindungsstrebe eine derartige Konstruktion aufweist.

In einer Ausführungsform verjüngt sich die Stärke, d.h. die Höhe der Schenkel im Bereich der Schenkelenden. So können die Schenkel von schräg oben in eine Aufnahme wie einen Schuh an einer korrespondierenden Stelle an der Lagerungsvorrichtung eingeführt werden, ohne dass das einführen behindert wird.

Die zentralen Arretierungsmittel sind an der Außenseite, d.h. an der vom Maskenmaterial abgewandten Seite der Schenkel angebracht. Die Mittel sind an der Außenfläche der Schenkel angebracht.

Bei den zentralen Arretierungsmitteln handelt es sich um je einen an der Außenfläche der Schenkel angebrachten Stift. Die Stifte sind bezogen auf die von den Schenkeln und der Verbindungsstrebe des Rahmens aufgespannten Fläche horizontal angeordnet.

Die Erfindung betrifft ferner eine Lagerungsvorrichtung gemäß Anspruch 10. Vorteilhafte Ausführungsformen ergeben sich aus den Unteransprüchen.

Demnach ist eine Lagerungsvorrichtung für den Kopf- und/oder Schulterbereich eines Patienten für die radiologische Diagnostik und/oder radioonkologische Behandlung vorgesehen. Die Lagerungsvorrichtung weist eine Basisplatte mit einem Aufnahmebereich und Arretierungsmitteln zur Aufnahme und Fixierung einer Maske mit einem U-förmigen Rahmen auf. Erfindungsgemäß weist die Basisplatte wenigstens ein zentrales Arretierungsmittel zur Fixierung des zentralen Bereichs wenigstens eines und vorzugsweise beider Schenkel des U-förmigen Rahmens auf.

Die Lagerungsvorrichtung eignet sich zur Aufnahme und Arretierung eines erfindungsgemäßen Rahmens.

In einer Ausführungsform ist der Aufnahmebereich eine U-förmige Vertiefung in der Basisplatte. Diese Vertiefung kann in Länge und Breite die Dimensionen der an der Basis zu fixierenden Maske und/oder eine geringere Tiefe als die an der Basis zu fixierende Maske aufweisen.

Die zentralen Arretierungsmittel sind an der Außenseite und/oder des Aufnahmebereichs angeordnet. So wird der zentrale Bereich der Schenkel des Rahmens an dessen Außenseite arretiert, welche für den Anwender besser zugänglich ist. Zudem wird die Lagerung des Kopfbereichs des Patienten nicht durch die zentralen Arretierungsmittel beeinträchtigt.

Die zentralen Arretierungsmittel der Basis sind Aufnahmen für die am Maskenrahmen angebrachte Stifte. Die Aufnahmen sind sind so ausgebildet, dass die Stifte durch Bewegung in der Ebene der Basisplatte in Richtung der Schenkelenden arretiert werden. Das Einführen der Stifte in die Öffnungen der Aufnahmen kann durch eine schräge Bewegung in Richtung der Basisplatte und der Schenkelenden erfolgen.

In einer Ausführungsform umfasst die Lagerungsvorrichtung ferner eine erfindungsgemäße Maske zur Fixierung des Kopf- und Nackenbereichs eines Patienten an der Basis. Die Maske kann reversibel an der Lagerungsvorrichtung fixiert sein.

In dieser Ausführungsform stellen die zentralen Arretierungsmittel der Basis stellen das Gegenstück der zentralen Arretierungsmittel des an der Basis fixierten Rahmens dar.

In einer Ausführungsform wird in der Arreststellung der zentralen Arretierungsmittel ein bezogen auf die Ebene der Basisplatte vertikales Abheben der zentralen Bereiche der Schenkel des Rahmens der Maske verhindert.

Weitere Einzelheiten und Vorteile der Erfindung ergeben sich aus dem nachfolgend anhand der Figuren beschriebenen Ausführungsbeispiel. In den Figuren zeigen:
- Figur 1:: eine erfindungsgemäße Maske in perspektivischer Ansicht,
- Figur 2:: Bauteile eines Rahmens einer erfindungsgemäßen Maske in Obenansicht,
- Figur 3:: eine Basis einer erfindungsgemäße Lagerungsvorrichtung in perspektivischer Ansicht (Figur 3a) und in Obenansicht (Figur 3b),
- Figur 4:: eine vergrößerte Ansicht eines zentralen Arretierungsmittels an der Basisplatte einer erfindungsgemäße Lagerungsvorrichtung in perspektivischer Ansicht als gelöstes Bauteil (Figur 4a) und im montierten Zustand (Figur 4b), und
- Figur 5:: eine Basis einer erfindungsgemäße Lagerungsvorrichtung mit einer darauf arretierten, erfindungsgemäßen Maske, und
- Figur 6:: eine vergrößerte Ansicht eines zentralen Arretierungsmittels einer erfindungsgemäße Lagerungsvorrichtung mit einem darin aufgenommenen zentralen Arretierungsmittel einer erfindungsgemäßen Maske.

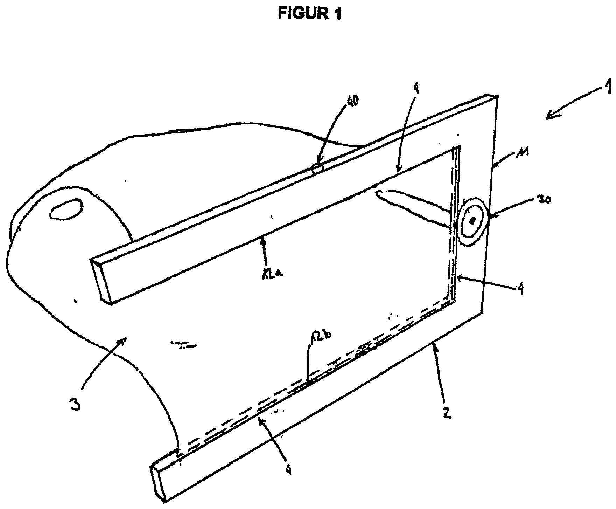

Figur 1 zeigt eine erfindungsgemäße Maske in perspektivischer Ansicht.

Die Maske 1 weist einen U-förmigen Rahmen 2 sowie ein bereits der Kopfform eines Patienten angepasstes Maskenmaterial 3 auf. Der Rahmen ist aus starrem Polycarbonat gefertigt, und das Maskenmaterial ist eine luftig gelochte Folie aus thermoplastischem Kunststoff.

Der U-förmige Rahmen 2 weist zwei Schenkel 12a, 12b sowie eine Verbindungsstrebe 11 auf. Die zwei Schenkel 12a und 12b sind über Verbindungsstrebe 11 miteinander verbunden, wobei die Verbindungsstrebe 11 im rechten Winkel auf sowohl Schenkel 12a als auch Schenkel 12b steht.

In der Mitte der Verbindungsstrebe 11 befindet sich ein Drehgelenk 30, wodurch die Schenkel 12a und 12b voneinander abgespreizt werden können. Eine Abspreizung der Schenkel 12a und 12b bietet dem Anwender insbesondere die Möglichkeit, beim Überziehen der Maske über den Kopf eines Patienten einen größeren Handlungsspielraum zu haben.

In den Bereichen 4 an ist das Maskenmaterial 3 mit dem Rahmen 2 verklebt und somit fest verbunden.

Die Maske 1 bestehend aus Rahmen 2 und Maskenmaterial 3 ist in deren Gesamtheit ein Einwegteil. Da der Rahmen 2 integral mit dem Maskenmaterial 3 verbunden ist, kann er nicht für weitere Patienten wiederverwendet werden, sondern wird zusammen mit dem patentenspezifisch angepassten Maskenmaterial entsorgt.

Der Rahmen ist nicht als Vollmaterial ausgeführt sondern in einer Strebenkonstruktion gefertigt.

An den Außenseiten beider Schenkel 12a und 12b befindet sich je ein Stift 40. Dieser ist in etwa in der längsseitigen Mitte der Schenkel an der Außenfläche angeordnet und steht im rechten Winkel zum Verlauf des Schenkels.

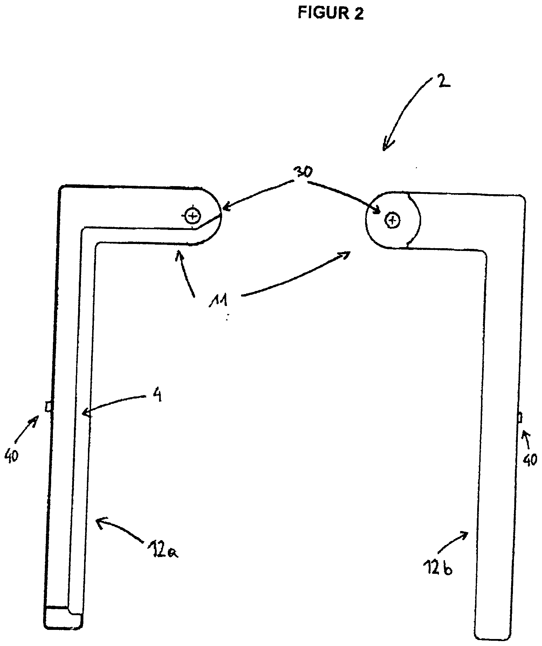

Figur 2 zeigt zwei L-förmige Bauteile eines erfindungsgemäßen Rahmens, wie er im Zusammenhang mit Figur 1 näher beschrieben wurde. Die Bauteile werden an dem Drehgelenk zu einem U-förmigen Rahmen zusammengesetzt.

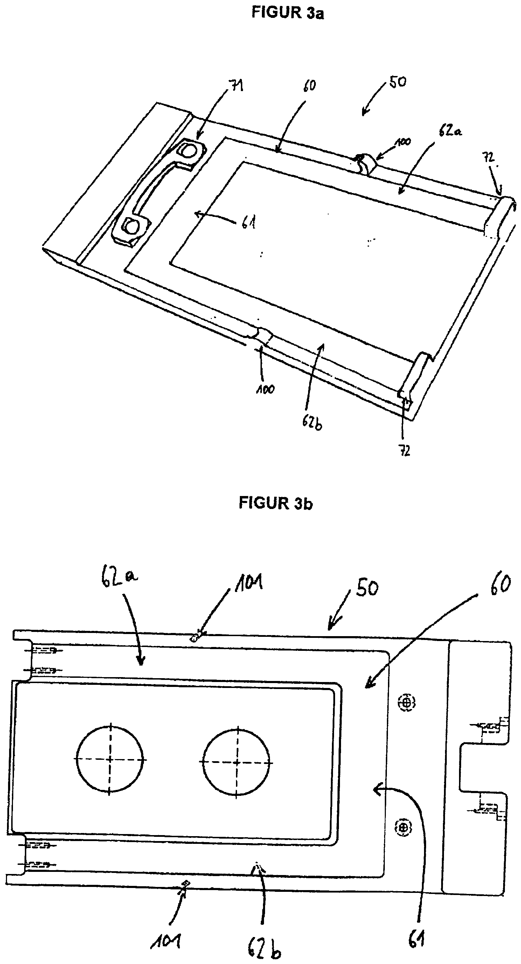

Figur 3a zeigt eine Basisplatte 50 einer erfindungsgemäßen Lagerungsvorrichtung in perspektivischer Ansicht. Figur 3b zeigt dieselbe Basisplatte in Obenansicht.

Diese weist an deren Oberfläche eine U-förmige Ausnehmung 60 mit zwei Schenkeln 62a und 62b und einer Verbindung 61 zwischen den Schenkeln auf. Außerhalb des zentralen Bereichs der Schenkel 62a, 62b der Ausnehmung 60 befinden sich Gewindebohrungen 101 zur Befestigung von zentralen Arretierungsmitteln 100.

Ferner weist die Basisplatte 50 mehrere Arretierungsvorrichtungen 71, 72 und100 zur Arretierung des Rahmens einer Maske auf.

Eine "obere", d.h. im Bereich der Verbindung 61 befindliche Arretierungsvorrichtung 71 ist ein zweiteiliger Riegel, der in der Ebene der Basisplatte 50 derart verschiebbar ist, dass in einer Stellung (geschlossen) Vorsprünge des Riegels einen Teil der Verbindung 61 der Ausnehmung überdecken.

Zwei "untere", d.h. im Endbereich der Schenkel 62a und 62b befindliche Arretierungsvorrichtungen 72 sind schuhartige Aufnahmen, die eine in Richtung der Schenkelenden weisende Öffnung besitzen. Ein in seiner Dicke verjüngter Endbereich der Schenkel 12a beziehungsweise 12b kann von schräg oben in die Öffnugnen des Schuhs eingeführt werden.

Zwei "zentrale" Arretierungsmittel 100 sind im zentralen Bereich der Ausnehmung 60 an deren Außenseite angeordnet.

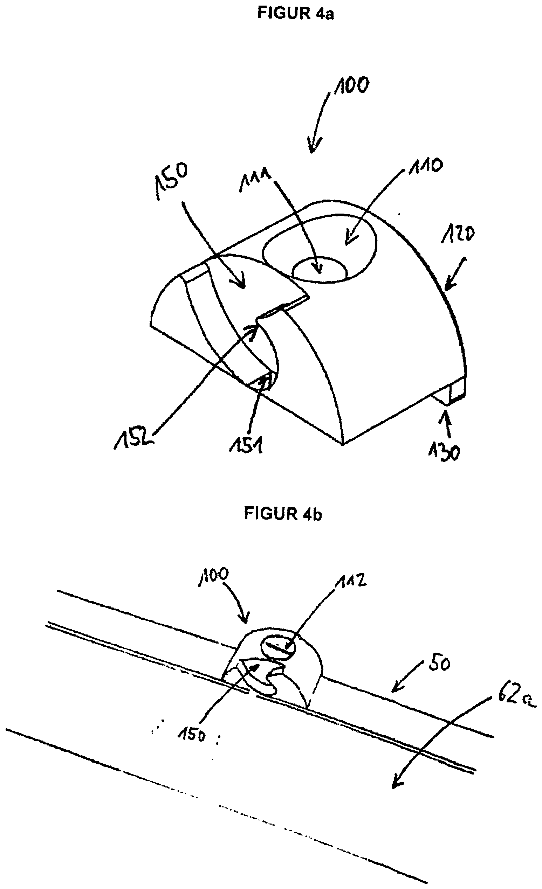

Figur 4a zeigt eine vergrößerte Ansicht eines dieser zentralen Arretierungsmittel 100 in perspektivischer Ansicht als gelöstes Bauteil. Figur 4b zeigt eine vergrößerte Ansicht desselben Arretierungsmittels 100 im an die Basisplatte 50 montierten Zustand.

Das zentrale Arretierungsmittel 100 ist in dieser Ausführungsform als Aufnahme ausgebildet. Es hat eine halbkreisförmige Gestalt, wobei das abgeschnittene Ende des Halbkreises an der Basisplatte 50 aufliegt. Im zentralen oberen Bereich weist das Bauteil eine Senkung 110 auf. Unterhalb der Senkung befindet sich eine das Bauteil durchdringende und am abgeschnittenen Ende des Halbkreises endende Bohrung 111. Das Arretierungsmittel ist mit Hilfe einer Schraube 112 an der Basisplatte 50 befestigt.

Das Bauteil ist rückseitig mit einer Fase 120 versehen, welche in deren Randbereich gegebenenfalls abgeschrägt sein kann. Über den unteren Rand der halbkreisförmigen Gestalt kann ein Fortsatz 130 hinausstehen, welcher eine einfachere Positionierung der Bohrung 111 über der Gewindeöffnung 101 bei der Montage ermöglicht.

An dessen Innenseite weist die Aufnahme 100 eine Ausnehmung 150 auf. Die Tiefe der Ausnehmung im Verhältnis zu der Innenoberfläche des Bauteils 100 ist konstant. Nach oben hin weist die Ausnehmung eine Öffnung auf, dies sich über deren volle Tiefe erstreckt. Die Ausnehmung weist einen abgerundeten Endbereich 151 auf, dessen Rundung so ausgebildet sein kann, dass sie formschlüssig an die Stifte 40 des Rahmens 2 angrenzt. Über die kopfseitige Öffnung des Bauteils 100 kann ein am Rahmen 2 befestigter Stift 40 in einem schrägen Winkel zur Ebene der Basisplatte 50 bis in den Endbereich 151 einschoben werden. Oberhalb des abgerundeten Endbereichs 151 der Ausnehmung 150 befindet sich ein Vorsprung 152, der eine vertikale Bewegung eines formschlüssig im Endbereich 151 aufgenommenen Stiftes 40 verhindert.

Das Arretieren einer Maske 1 mit einem U-förmigen Rahmens 2 an der gezeigten Basisplatte 50 erfolgt durch
(i) in Richtung des Schenkels 62a und im Verhältnis zur Ebene der Basisplatte 50 leicht schräg einfallendes Einstecken der verjüngten Schenkelenden 12a bzw. 12b in die Aufnahmen der Schuhe 72 an der Basisplatte,
(ii) gleichzeitiges Einstecken der Stifte 40 in die kopfseitigen Öffnungen der Aufnahmen 100,
(iii) anschließendes Schieben des Rahmens 2, bis die Enden der Schenkel 12a und 12b am Anschlag der Aufnahmen der Schuhe 72 und die Stifte 40 im Endbereich 151 der Aufnahmen 100 aufgenommen sind,
(iv) dabei gleichzeitiges Senken des Rahmens 2 in die formschlüssige U-förmige Ausnehmung 60 der Basisplatte 50,
(v) finale Arretierung des Rahmens 2 durch Verschieben des Riegels 71 in dessen Schließstellung, sodass die Vorsprünge des Riegels 71 Teile der Strebe 11 überdecken.

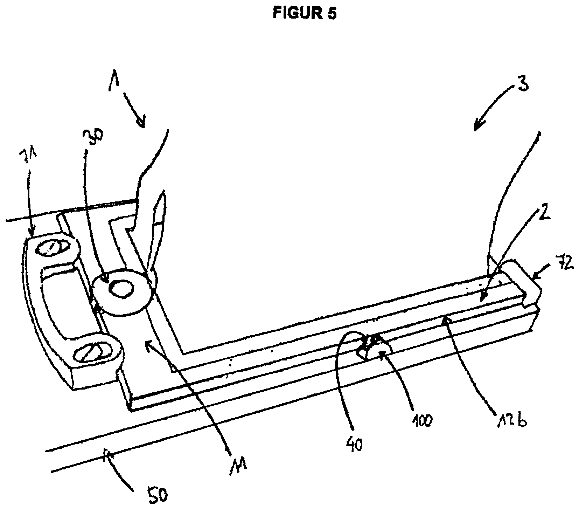

Figur 5 zeigt eine Basis 50 einer erfindungsgemäße Lagerungsvorrichtung mit einer darauf arretierten, erfindungsgemäßen Maske 1.

Basisplatte 50 und Maske 1 an sich wurden bereits im Zusammenhang mit den vorangehenden Figuren beschrieben.

Der Rahmen 2 wird durch die Arretierungsmittel 71, 72, 100 in der mit dem Rahmen formschlüssigen Ausnehmung 60 gehalten. Die Schenkelenden 12a und 12b sind in Schuhe 72 eingesteckt, die Stifte 40 sind in Aufnahmen 100 eingesteckt, und der Riegel 71 befindet sich in geschlossener Position, wobei ein Teil der Verbindungsstrebe 11 des Rahmens 2 von den Vorsprüngen des Riegels 71 überdeckt werden. Weder ein vertikales Abheben der Schenkelenden aus Schuhen 72, noch ein vertikales Abheben der Stifte 40 aus den Aufnahmen 100 (und damit kein vertikales Abheben der zentralen Bereiche der Schenkel von der Basisplatte 50), noch ein vertikales Abheben der Verbindungsstrebe 11 über die Vorsprünge des Riegels 71 ist möglich.

Das Drehgelenk 30 des Rahmens 2 ist im arretierten Zustand nicht abgewinkelt und die Schenkel 12a und 12b des Rahmens stehen parallel zueinander.

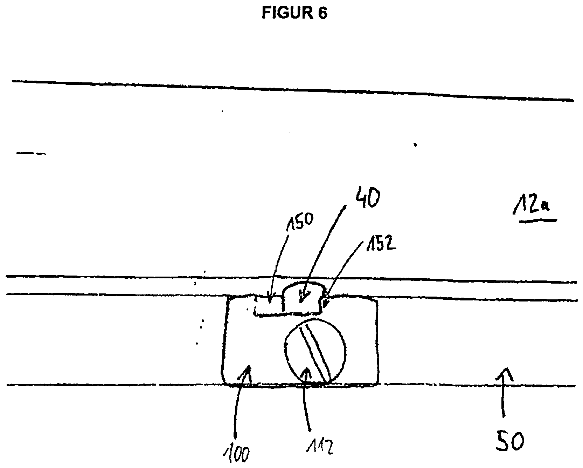

Figur 6 zeigt eine Vergrößerung der Aufnahme 100 mit einem darin aufgenommenen, an der Außenfläche des Schenkels 12a angebrachten Stift 40.

Die Aufnahme 100 wurde bereits anhand der Diskussion von Figuren 4a und 4b genauer beschrieben. Ein Entkommen des Stiftes 40 in vertikaler Richtung aus der Ausnehmung 150 ist durch den Vorsprung 152 oberhalb des Endbereichs 151 der Ausnehmung 150 nicht möglich.

Zusammenfassend ergibt sich, dass mit Hilfe einer erfindungsgemäßen Maske und mit Hilfe einer erfindungsgemäßen Lagerungsvorrichtung eine fehlerhafte Einfassung und Befestigung des Maskenmaterials im Rahmen durch den Anwender ausgeschlossen werden kann, und signifikanten Kostenfolgen oder Lieferungsverzögerungen durch Beschädigungen am Rahmen entgegengewirkt werden kann. Dennoch wird eine präzise Lagerung des Kopf- und Halsbereichs von Patienten in der Diagnose und radioonkologischen Behandlung erreicht, wobei durch die in der Basis eingerastete Maske jegliche Drehbewegung des Kopfes in allen drei Achsen wirkungsvoll verhindert wird.

## Patentansprüche

1. Maske (1) zur Fixierung des Kopf- und/oder Schulterbereichs eines Patienten an einer Lagerungsvorrichtung für die radiologische Diagnostik und/oder radioonkologische Behandlung, wobei die Maske (1) einen U-förmig gestalteten Rahmen (2) mit zwei Schenkeln (12a, 12b) und einer Verbindungsstrebe (11) und ein thermoplastisches Maskenmaterial (3) aufweist, wobei der Rahmen (2) und das Maskenmaterial (3) ein integrales Bauteil darstellen und irreversibel miteinander verbunden sind, wobei sich an der Außenseite beider Schenkel (12a, 12b) je ein bezogen auf die von den Schenkeln und der Verbindungsstrebe des Rahmens aufgespannten Fläche horizontal angeordneter Stift (40) befindet, der etwa in der längsseitigen Mitte der Schenkel (12a, 12b) an der Außenfläche angeordnet ist, und im rechten Winkel zum Verlauf des Schenkels steht.

2. Maske gemäß Anspruch 1, **dadurch gekennzeichnet, dass** es sich bei der Maske (1) um ein Einwegteil handelt.

3. Maske gemäß einem der vorhergehenden Ansprüche, **dadurch gekennzeichnet, dass** die Verbindungsstrebe (11) wenigstens ein Gelenk (30) aufweist, mittels dessen die Schenkel (12a, 12b) des Rahmens (2) aufspreizbar sind.

4. Maske gemäß Anspruch 3, **dadurch gekennzeichnet, dass** das Gelenk (30) in der Mitte der Verbindungsstrebe (11) angeordnet ist.

5. Maske gemäß einem der vorangehenden Ansprüche, **dadurch gekennzeichnet, dass** der Rahmen (2) wenigstens teilweise aus Polycarbonat gefertigt ist.

6. Maske gemäß einem der vorangehenden Ansprüche, **dadurch gekennzeichnet, dass** der Rahmen (2) zumindest abschnittsweise eine Streben- und/oder Fächerkonstruktion aufweist.

7. Maske gemäß einem der vorangehenden Ansprüche, **dadurch gekennzeichnet, dass** sich die Stärke des Rahmens (2) an den Enden der Schenkel (12a, 12b) verjüngt.

8. Lagerungsvorrichtung für den Kopf- und/oder Schulterbereich eines Patienten für die radiologische Diagnostik und/oder radioonkologische Behandlung mit einer Basisplatte (50) und einer reversibel daran fixierten Maske (1) gemäß einem der vorhergehenden Ansprüche, wobei die Basisplatte (50) einen Aufnahmebereich (60) mit Arretierungsmitteln (71, 72, 100) zur Aufnahme und Fixierung der Maske (1) umfasst, wobei die Basisplatte (50) an der Außenseite des Aufnahmebereichs (60) Aufnahmen (100) für die Stifte (40) an der Außenfläche der Schenkel (12a, 12b) des U-förmigen Rahmens (2) zur Fixierung des zentralen Bereichs der Schenkel (12a, 12b) aufweist, wobei die Aufnahmen (100) so ausgebildet sind, dass die Stifte (40) durch eine Bewegung in Richtung der Schenkelenden darin arretiert werden können.

9. Lagerungsvorrichtung gemäß Anspruch 8, **dadurch gekennzeichnet, dass** der Aufnahmebereich (60) als eine U-förmige Vertiefung ausgebildet ist.

10. Lagerungsvorrichtung gemäß Anspruch 8 oder 9, **dadurch gekennzeichnet, dass** in der Arreststellung der Stifte (40) und Aufnahmen (100) ein bezogen auf die Ebene der Basisplatte (50) vertikales Abheben der zentralen Bereiche der Schenkel (12a, 12b) verhindert wird.

## Claims

1. A mask (1) for fixing the head and/or shoulder region of a patient to a positioning apparatus for radiological diagnosis and/or radiooncological treatment, wherein the mask (1) has a frame (2) designed in U shape and having two limbs (12a, 12b) and a connection strut (11) and has a thermoplastic mask material (3), with the frame (2) and the mask material (3) representing an integral component and being irreversibly connected to one another; and wherein a respective pin (40) is located at the outer side of both limbs (12a, 12b) that is arranged horizontally with respect to the surface spanned by the limbs and by the connection strut of the frame, that is arranged at the outer surface approximately at the longitudinal center of the limbs (12a, 12b), and that is at a right angle to the extent of the limb.

2. A mask in accordance with claim 1, **characterized in that** the mask (1) is a disposable part.

3. A mask in accordance with one of the preceding claims, **characterized in that** the connection strut (11) has at least one joint (30) by means of which the limbs (12a, 12b) of the frame (2) can be spread apart.

4. A mask in accordance with claim 3, **characterized in that** the joint (30) is arranged at the center of the connection strut (11).

5. A mask in accordance with one of the preceding claims, **characterized in that** the frame (2) is produced at least partly of polycarbonate.

6. A mask in accordance with one of the preceding claims, **characterized in that** the frame (2) has a strut design and/or a fan design at least sectionally.

7. A mask in accordance with one of the preceding claims, **characterized in that** the thickness of the frame (2) tapers at the ends of the limbs (12a, 12b).

8. A positioning apparatus for the head and/or shoulder region of a patient for radiological diagnosis and/or radiooncological treatment having a base plate (50) and a mask (1) in accordance with one of the preceding claims reversibly fixed thereto, wherein the base plate (50) comprises a reception region (60) having locking means (71, 72, 100) for receiving and fixing the mask (1), with the base plate (50) having receivers (100) for the pins (40) at the outer surface of the limbs (12a, 12b) of the U-shaped frame (2) at the outer side of the reception region (60) for fixing the central region of the limbs (12a, 12b) and with the receivers (100) being configured such that the pins (40) can be locked in the limb ends by a movement in the direction of said limb ends.

9. A positioning apparatus in accordance with claim 8, **characterized in that** the reception region (60) is configured as a U-shaped recess.

10. A positioning apparatus in accordance with claim 8 or clam 9, **characterized in that** a raising of the central regions of the limbs (12a, 12b) that is vertical with respect to the plane of the base plate (50) is prevented in the locked position of the pins (40) and receivers (100).

## Revendications

1. Masque (1) destiné à la fixation de la zone de la tête et/ou des épaules d'un patient à un dispositif de positionnement pour le diagnostic radiologique et/ou le traitement radio-oncologique, dans lequel
le masque (1) comporte un cadre (2) conçu en forme de U doté de deux branches (12a, 12b) et d'une barre de liaison (11) et un matériau thermoplastique faisant office de masque (3), le cadre (2) et le matériau faisant office de masque (3) représentant un élément intégral et étant reliés l'un à l'autre de manière irréversible, dans lequel
une tige (40) disposée de manière horizontale par rapport à la surface déployée par les branches et la barre de liaison du cadre se trouve respectivement sur le côté extérieur des deux branches (12a, 12b), laquelle tige est disposée sur la surface extérieure approximativement au milieu des branches (12a, 12b) dans le sens de la longueur et forme un angle droit par rapport à la trajectoire des branches.

2. Masque selon la revendication 1, **caractérisé en ce que** le masque (1) est une pièce à usage unique.

3. Masque selon l'une des revendications précédentes, **caractérisé en ce que** la barre de liaison (11) comporte au moins une articulation (30), au moyen de laquelle les branches (12a, 12b) du cadre (2) peuvent être écartées.

4. Masque selon la revendication 3, **caractérisé en ce que** l'articulation (30) est disposée au milieu de la barre de liaison (11).

5. Masque selon l'une des revendications précédentes, **caractérisé en ce que** le cadre (2) est fabriqué au moins partiellement en polycarbonate.

6. Masque selon l'une des revendications précédentes, **caractérisé en ce que** le cadre (2) comporte au moins sur certaines parties une construction en treillis et/ou en éventail.

7. Masque selon l'une des revendications précédentes, **caractérisé en ce que** l'épaisseur du cadre (2) s'amincit aux extrémités des branches (12a, 12b).

8. Dispositif de positionnement pour la zone de la tête et/ou des épaules d'un patient pour le diagnostic radiologique et/ou le traitement radio-oncologique comprenant une plaque de base (50) et un masque (1) selon l'une des revendications précédentes fixé à celle-ci de manière réversible, dans lequel la plaque de base (50) comprend une zone de réception (60) dotée de moyens de blocage (71, 72, 100) pour recevoir et fixer le masque (1), la plaque de base (50) comportant sur le côté extérieur de la zone de réception (60) des logements (100) pour les tiges (40) de la surface extérieure des branches (12a, 12b) du cadre (2) en forme de U, destinés à fixer la zone centrale des branches (12a, 12b), dans lequel les logements (100) sont réalisés de telle manière que les tiges (40) peuvent être bloquées dans ceux-ci par un déplacement en direction des extrémités de branche.

9. Dispositif de positionnement selon la revendication 8, **caractérisé en ce que** la zone de réception (60) est réalisée en tant que creux en forme de U.

10. Dispositif de positionnement selon la revendication 8 ou 9, **caractérisé en ce que**, dans la position de blocage des tiges (40) et des logements (100), un soulèvement vertical de la zone centrale des branches (12a, 12b) par rapport au niveau de la plaque de base (50) est empêché.
